# EUROPEAN PATENT APPLICATION

(11) **EP 4 023 207 A1**
(43) Date of publication of application: **06.07.2022**
(21) Application number: 20859120.6
(22) Date of filing: 20.08.2020
(51) Int. Cl.: A61K 8/19, A61Q 1/12, C01B 21/064

(54) **MODIFIED BORON NITRIDE POWDER**

(30) Priority: 28.08.2019 JP 2019155860
(71) Applicant: Tokuyama Corporation, Shunan-shi, Yamaguchi 745-8648 (JP)
(72) Inventor: ABURATANI, Makoto, Shunan-shi, Yamaguchi 745-8648 (JP)
(74) Representative: J A Kemp LLP
(86) International application number: PCT/JP2020/031459
(87) International publication number: WO 2021/039586

(57) **Abstract**

A boron nitride powder having a voltage density of not less than +1 V/g as measured by a triboelectric charging test.

## Description

### Technical Field:

The present invention relates to a modified boron nitride powder whose properties have been modified to be suitable for cosmetic use. The present invention further relates to a method for modifying a boron nitride powder.

### Background Art:

Boron nitride power, which is a white extender pigment with a layered hexagonal crystal structure, has been used widely as a powder base material for cosmetics such as makeup cosmetics for the purpose of giving a gloss and an improved feeling and increasing quantity, for example.

Meanwhile, cosmetics need to be excellent in covering properties, transparency, spreadability, lubricity, adherence to human skin, and the like. Boron nitride power is known for its properties of imparting especially spreadability and adherence. In an attempt to further improve these properties, attention is given to the shape of primary particles, such as the particle diameter (long diameter), the thickness, and the aspect ratio. For example, Patent Document 1 discloses that a boron nitride power of primary particles with an average long diameter of 5 to 8 µm, an average thickness of 0.25 to 0.5 µm, and an average aspect ratio (long diameter/thickness) of 15 to 20 is excellent in imparting spreadability and adherence.

However, there is a limit to improving spreadability and adherence only by adjusting the shape of primary particles of a boron nitride powder.

Further, Patent Document 2 discloses modifying a boron nitride powder in the following manner: A hexagonal boron nitride powder having a graphitization index (GI) of not more than 2.0 and a specific surface area of not more than 15 m²/g is washed with an aqueous solution of 0.02 to 0.5 chemical equivalents of acid per mole of the hexagonal boron nitride, followed by drying; and the powder thus washed and dried is kept in an airtight container so that it has no contact with carbon, and heat treated at 1800°C to 1950°C for 1 to 5 hours in a nitrogen atmosphere. The thus-obtained modified boron nitride powder achieves an improved whiteness of 95.0 or more, but is still unable to impart sufficient spreadability and adherence required for cosmetic use.

### Prior Art Documents:

### Patent Documents:

Patent Document 1: JP 6109466 B2
Patent Document 2: JP 2004-35273 A

### Summary of the Invention:

### Problems to be solved by the invention:

An object of the present invention is to provide a modified boron nitride powder capable of significantly improving properties required for corsmetics, such as spreadability and adherence, when blended in cosmetics.

Another object of the present invention is to provide a modification method for obtaining the modified boron nitride powder.

### Means for solving the problems:

The present inventors have contemplated that a conventional boron nitride powder contains a large amount of lumps (aggregates that are large enough to be seen visually), which tend to cause unevenness when put on hand or a cosmetic sponge, and identified these lumps as a cause of insufficient spreadability and adherence for cosmetic use. A large number of repeated experiments that have been conducted based on this idea have finally led to the following novel finding. That is, a boron nitride powder containing a large amount of lumps exhibits negative chargeability when subjected to a triboelectric charging test by using a rotating drum that is made of alumite treated aluminum and includes windows made of boron silicate glass. By heat treating this powder under specific conditions, it is modified to have positive triboelectric chargeability, which results in reduced lumps. The present invention has been completed based on this finding.

The present invention provides a modified boron nitride powder having a voltage density of not less than +1 V/g as measured by a triboelectric charging test.

In the modified boron nitride powder of the present invention, it is suitable that:
(1) the modified boron nitride powder has a voltage density of not less than +10 V/g as measured by a triboelectric charging test;
(2) the modified boron nitride powder has an avalanche energy of not more than 40 mJ/kg;
(3) the modified boron nitride powder contains primary particles with an average long diameter in a range of 2 to 20 µm, an average thickness in a range of 0.2 to 2.0 µm, and an average aspect ratio in a range of 5 to 20; and
(4) the modified boron nitride powder is for use as a compounding agent for cosmetics.

The present invention further provides a method for modifying a boron nitride powder, comprising: heat treating a negatively triboelectrically charged boron nitride powder (hereinafter, may be referred to simply as a negatively charged boron nitride powder) under a flow of an inert gas at a temperature of 1300°C to 2200°C until it has a voltage density of not less than +1 V/g as measured by a triboelectric charging test.

In the method of the present invention, it is preferable that:
(1) the heat treatment is performed until the negatively charged boron nitride powder has a voltage density of not less than +10 V/g as measured by the triboelectric charging test; and
(2) the heat treatment is performed while the negatively charged boron nitride powder is fluidized under the flow of the inert gas.

In the present invention, the triboelectric charging test is performed by using a rotating drum (drum capacity: 332 cc) that is made of alumite treated aluminum and includes windows made of boron silicate glass. As described in Examples below, a sample powder is put in the rotating drum, which is then rotated at a rate of 10 rpm for 300 seconds, so that the sample powder is triboelectrically charged (see Examples for detailed conditions) . Thus, the chargeability of a boron nitride powder in the present invention refers to triboelectric chargeability as measured by using the rotating drum type powder flowability measurement device as described above.

Further, avalanche energy as mentioned above is measured by performing a powder flowability test using also the aforementioned powder flowability measurement device.

### Effects of the invention:

It is proved by the above-described triboelectric charging test that the modified boron nitride powder of the present invention has properties such that it is positively charged, while a conventional boron nitride powder is negatively charged. Namely, the modified boron nitride powder of the present invention, due to its positive chargeability, achieves a fluffy texture (i.e., a lightweight texture) with extremely few lumps.

Thus, the modified boron nitride powder of the present invention, when used in cosmetics such as a powder foundation, can significantly improve spreadability and adherence, enabling the powder foundation to adhere to the surface of skin evenly by a single application operation.

In the present invention, in order to obtain the above-described modified boron nitride powder that is triboelectrically charged positively, it is necessary to heat treat a conventionally known boron nitride powder (i. e. , a boron nitride powder that is triboelectrically charged negatively) under the flow of an inert gas at a temperature of 1300°C to 2200°C.

Various methods are known for producing a boron nitride powder. A powder produced by any method contains particles on the surface of which an extremely slight amount of functional groups such as OH groups and CN groups and/or impurities such as B₂O₃ are present. Such a boron nitrite powder containing particles on the surface of which functional groups and/or impurities are present is triboelectrically charged negatively when a rotating drum made of a predetermined material is rotated. In view of this, the heat treatment is performed in the present invention to remove the functional groups and/or impurities. As a result, the heat treated boron nitride powder (modified boron nitride powder) exhibits positive chargeability.

For example, Patent Document 2 above also discloses heat treating a boron nitride powder. However, this heat treatment is performed in a nitrogen atmosphere in an airtight container. In contrast, the heat treatment of the present invention is performed under the flow of an inert gas, i.e., under so-called dynamic conditions, thereby removing the substances present on the particle surface that contribute to negative chargeability (hereinafter, may be referred to simply as negative charge substances). In Patent Document 2, the heat treatment is performed simply in a nitrogen gas atmosphere under static conditions with no nitrogen gas flowing. Such a heat treatment does not remove the negative charge substances on the particle surface and, thus, cannot achieve a positively charged boron nitride powder. In fact, as proved by Examples below, a heat treatment in each of Examples 1 to 11 performed under dynamic conditions with an inert gas (a nitrogen gas or an Ar gas) flowing achieves a voltage density of not less than +11 V/g, while a heat treatment in Comparative Example 3 performed simply in a nitrogen atmosphere under static conditions with no nitrogen gas flowing results in a voltage density of -6 V/g and fails to achieve a positively charged boron nitride powder.

Further, a description is given of a case where boron nitride is produced by a reduction nitridation method. For example, a boron source compound is heated to a temperature of approximately 1300°C to 1550°C in a nitriding furnace into which a nitrogen gas is being fed, so that the boron source compound is reduced and nitrided to form amorphous boron nitride. Then, the amorphous boron nitride is heat treated at a temperature of approximately 1700°C to 2200°C to be acceleratedly crystallized, resulting in a crystallized boron nitride powder. In this case, since the heat treatment (crystallization) is performed in the same nitriding furnace as the preceding reduction-nitridation, this heat treatment is performed in the same manner as in the present invention. However, the boron nitride powder obtained in this case is triboelectrically charged positively but still lumpy. This seems to be because the boron nitride powder at this stage contains a large amount of impurities such as alkali substances and B₂O₃, which cannot be removed by the heat treatment under the flow of a nitrogen gas (inert gas).

In the present invention, the heat treatment (modification treatment) for changing triboelectric chargeability from negative to positive is preceded by at least acid washing and water washing after the crystallization of boron nitride. This allows most (not all) of the impurities that contribute to negative chargeability to be removed. Thus, the heat treatment under the flow of an inert gas can remove a slight amount of the remaining impurities, thereby obtaining a positively triboelectrically charged boron nitride powder with reduced lumps.

### Mode for Carrying Out the Invention:

### <Modified boron nitride powder>

A modified boron nitride powder of the present invention, which contains hexagonal boron nitride particles, has a voltage density (charge amount) of not less than +1 V/g, preferably not less than +10 V/g, and more preferably not less than +30 V/g as measured by a triboelectric charging test using a rotating drum type powder flowability measurement device (see Examples for detailed conditions) . A boron nitrite powder with a higher voltage density contains less aggregated particles and, thus, is fluffy with few lumps. Such a boron nitride powder contributes to improving spreadability and adherence when blended in cosmetics. A boron nitride powder with a voltage density of less than 1 V/g (or with negative chargeability) cannot improve spreadability and adherence of cosmetics because particles tend to aggregate to form lumps. The upper limit of the voltage density is not particularly limited. However, a boron nitride powder that is likely to be excessively positively charged may be difficult to handle. On this account, the voltage density is preferably not more than +200 V/g, more preferably not more than +100 V/g.

In other words, a heat treatment for modification as described below is performed until the charge density falls within the aforementioned range.

The modified boron nitride powder of the present invention exhibits excellent flowability due to its positive triboelectric chargeability as described above. The flowability can be represented by avalanche energy (change in potential energy before and after an avalanche) that indicates powder flowability. More specifically, avalanche energy is measured by using a rotating drum type powder flowability measurement device (see Examples for detailed conditions) as used for measuring the voltage density. The lower the value is, the more excellent the flowability is, which contributes to achieving good spreadability and adherence. For example, the modified boron nitride powder of the present invention has an avalanche energy in a range of not more than 40 mJ/kg, preferably not more than 35 mJ/kg, and more preferably not more than 30 mJ/kg. There is no lower limit of the avalanche energy, though it is usually not less than 10 mJ/kg.

The modified boron nitride powder of the present invention is not particularly limited as to properties other than the charge density and the flowability based on the charge density, but desirably has properties conventionally required for cosmetic use.

For example, primary particles of the modified boron nitride powder preferably have an average long diameter in a range of 2 to 20 µm, particularly 3 to 10 µm, an average thickness in a range of 0.2 to 2.0 µm, particularly 0.3 to 1.0 µm, and an average aspect ratio (average long diameter/average thickness) in a range of 5 to 20, particularly 6 to 18. Further, the median diameter (D1: volume-based median diameter D₅₀) as measured by a laser diffraction scattering method is preferably 4 to 30 µm. Furthermore, the median diameter (D2) as measured by a laser diffraction scattering method in a state where the powder is ultrasonically dispersed in ethanol is preferably 4 to 14 µm.

Further, the amount of boron eluted in the modified boron nitride powder of the present invention is preferably not more than 20 ppm. The amount of eluted boron is measured by a method determined by the Japanese Standards of Quasi-drug Ingredients 2006. It is specified in the Japanese Standards of Quasi-drug Ingredients 2006 that the amount of eluted boron should be not more than 20 ppm from a safety and hygiene perspective.

Further, the modified boron nitride powder of the present invention generally has a whiteness (L value) in a range of not less than 90.0, a redness (a value) in a range of -3.0 to 0, and a yellowness (b value) in a range of 0 to 15.0 in the Lab color system.

Since the above-described modified boron nitride powder of the present invention contains few lumps, it has a low bulk density. The bulk density varies greatly depending on the average long diameter and the average thickness. For example, when the average long diameter is 4 µm, and the average thickness is approximately 0.6 µm, the light bulk density is not more than 0.11 g/cm³, and the tapped bulk density is not more than 0.33 g/cm³. When the average long diameter is 9 µm, and the average thickness is approximately 1 µm, the light bulk density is not more than 0.2 g/cm³, and the tapped bulk density is not more than 0.6 g/cm³.

A fluidization test using a rotating drum type powder flowability measurement device as used for measuring the charge density and the triboelectric avalanche energy can prove that the modified boron nitride powder of the present invention is fluffy with few lumps. More specifically, the fluidization test (see Examples for detailed conditions) is performed in the following manner: A standard rotating drum containing a 100 cc sample powder is rotated rapidly; and the height of a lower part of the powder layer in the drum is measured. The height of the powder layer of the modified boron nitride powder of the present invention is not less than 2.2 cm at a rotation frequency of the drum of 20 rpm, and not less than 2.8 cm at 50 rpm. The higher this value is, the fluffier the powder is with few lumps.

Basic flowability energy in a dynamic flowability test is known as a parameter indicating how fluffy the powder is with few aggregates. As described in Examples below, a blade of a powder rheometer (e.g., FT-4 manufactured by Malvern Instruments Ltd.) is inserted in a 160 mL split container filled with a sample powder, and torque that acts on this blade represents the basic flowability energy. The lower the basic flowability energy (torque acting on the blade) is, the fluffier the powder is with few lumps. The boron nitride powder of the present invention has a basic flowability energy of not more than 100 mJ, which proves that the powder is fluffy with few lumps (aggregates).

### <Production of boron nitride powder>

A boron nitride powder to be modified in the present invention can be produced by any of various methods known per se. Known examples of typical production methods include a melamine method and a reduction nitridation method. As described above, a powder obtained by any of these methods contains particles on the surface of which functional groups such as OH and CN and/or impurities such as B₂O₃ are present. These substances cannot be removed completely by acid washing and the like, causing the resultant powder to be triboelectrically charged negatively under predetermined conditions.

For example, the melamine method is performed in the following manner. Boric acid and melamine are allowed to react with each other at a temperature of approximately 1000 °C to form a low crystallinity boron nitride powder, which is then heated at a temperature of approximately 1700°C to 2200°C to be highly crystallized. Further, by-products other than the boron nitride present in the reaction product are washed and removed.

The reduction nitridation method is performed in the following manner. An oxygen-containing boron compound, a carbon source, and an oxygen-containing alkaline earth metal compound are heated in a nitrogen atmosphere, so that they are reduced and nitrided to form amorphous boron nitride. Then, the amorphous boron nitride is further heated at a higher temperature to be crystallized, resulting in a hexagonal boron nitride powder. Thereafter, by-products other than the boron nitride present in the reaction product are removed by acid washing.

In the present invention, either method may be used to produce a boron nitride powder. The melamine method takes a long time for the heating process, and often requires intermediate or posttreatment process for crushing, pulverization, classification and the like, resulting in a high production cost. In contrast, the reduction nitridation method takes a short time for the heating process, and requires fewer steps in intermediate or posttreatment process, resulting in a lower production cost. In addition, primary particles of the boron nitride powder obtained by the reduction nitridation method inherently aggregate less strongly and are uniform in diameter. Thus, this boron nitride powder requires less labor for crushing, pulverization, classification and the like, and is advantageous in achieving a primary particle shape especially suitable for cosmetic use. Moreover, this boron nitride powder has the advantage at the modification that is remarkable effect by a heat treatment to be detailed below. For these reasons, the reduction nitridation method is suitably used in the present invention.

### <Production of boron nitride powder by reduction nitridation method>

Hereinafter, a description will be given of a method for producing a boron nitride powder by the reduction nitridation method.

As described above, this method uses, as raw materials, an oxygen-containing boron compound, a carbon source, and an oxygen-containing alkaline earth metal compound. These raw materials are mixed to react with each other in a nitrogen atmosphere (reduction and nitridation), followed by removal of by-products and/or impurities.

### Oxygen-containing boron compound:

The oxygen-containing boron compound used as a boron source is not particularly limited. Examples include boric acid, boric anhydride, metabolic acid, perboric acid, hypoboric acid, sodium tetraborate, and sodium perborate. Usually, boric acid, which is easily available, is suitably used.

### Carbon source:

The carbon source is not particularly limited. Examples include: amorphous carbon such as carbon black, activated carbon, or carbon fiber; crystalline carbon such as diamond, graphite, or nanocarbon; and pyrolytic carbon obtained by pyrolyzing a monomer or a polymer. Usually, carbon black, which is inexpensive, is used.

The carbon source functions as an agent for reducing the oxygen-containing boron compound. When the carbon source is added in a larger amount, the boron nitride powder is obtained in a higher yield. However, an excessive addition of the carbon source causes the carbon to remain as an impurity. On the other hand, when the carbon source is added in a smaller amount, the boron nitride powder is obtained in a lower yield. Although the addition amount of the carbon source is not particularly limited, it is preferably set so that the element ratio (B/C) of the oxygen-containing boron compound to the carbon source is 0.5 to 1.0.

### Oxygen-containing alkaline earth metal compound:

The oxygen-containing alkaline earth metal compound functions as a crystallization catalyst. The oxygen-containing alkaline earth metal compound for this purpose is not particularly limited. Examples include magnesium oxide, calcium oxide, magnesium carbonate, calcium carbonate, magnesium hydrogen carbonate, calcium hydrogen carbonate, magnesium hydroxide, calcium hydroxide, magnesium nitrate, calcium nitrate, magnesium sulfate, calcium sulfate, magnesium phosphate, calcium phosphate, magnesium oxalate, and calcium oxalate. Two or more of these compounds may be used in combination.

When the oxygen-containing alkaline earth metal compound is added in a larger amount, the resultant boron nitride particles have a larger diameter. When the oxygen-containing alkaline earth metal compound is added in a smaller amount, the resultant boron nitride particles have a smaller diameter. Thus, the diameter of the boron nitride particles can be adjusted suitably based on this addition amount. In order to obtain the boron nitride powder containing primary particles with the aforementioned shape, it is usually preferable to add the oxygen-containing alkaline earth metal compound so that the molar ratio (MO/B₂O₃) of the oxygen-containing alkaline earth metal compound (MO; M is an alkaline earth metal) to the oxygen-containing boron compound (B₂O₃) in terms of oxide is in a range of 0.01 to 1.00.

### Mixing of raw materials:

The above-described raw materials (i.e., the oxygen-containing boron compound, the carbon source, and the oxygen-containing alkaline earth metal compound) are mixed in a manner not particularly limited, by using a common mixer such as a vibrating mill, a bead mill, a ball mill, a Henschel mixer, a drum mixer, a vibrating agitator, or a V-shaped mixer.

### Reduction and nitridation:

The reduction and nitridation using the aforementioned raw material mixture are performed in the following manner: The raw material mixture is supplied in a nitriding furnace and heated in a nitrogen atmosphere, whereby the oxygen/boron-containing compound is reduced and nitrided to form boron nitride.

This reaction proceeds at a temperature of not less than 1200°C and is represented by the following formula.

B₂O₃ + 3C + N₂ → 2BN + 3CO

The reduction and nitridation result in amorphous boron nitride, which is further heated at a higher temperature, e.g., not less than 1700°C, to be crystallized, resulting in a hexagonal boron nitride powder.

The reduction nitridation reaction proceeds at a temperature heated to not less than 1200°C, particularly not less than 1300°C, and the heating is continued even after the formation of the boron nitride, which allows the boron nitride to be crystallized at a high temperature of not less than 1700°C. Upon the heating, it is important to control the amount of carbon present in the reaction product. Specifically, the reaction preferably proceeds in a nitrogen atmosphere so that the carbon concentration is reduced to not more than 5 mass% by the time the temperature reaches 1550°C. When the carbon remains in an amount of more than 5 mass%, black impurities such as CaB₆ are generated. The carbon concentration of the reaction mixture can be controlled by using an X-ray fluorescence spectrometer, and a carbon concentration of as low as approximately 1 mass% is industrially acceptable.

It is not particularly limited how to confirm whether the carbon concentration of the reactant falls within the range of not more than 5 mass% by the time the temperature reaches 1550°C. For example, the carbon amount may be controlled directly by measuring the carbon concentration of the reactant at a temperature of 1550°C. Alternatively, it is industrially suitable to control the carbon amount in the following manner. That is, a preliminary experiment is conducted to specify the time taken to reduce the carbon concentration of the reactant to not more than 5 mass% under the operating conditions of the nitriding furnace including the aforementioned temperature profile, and the carbon amount may be controlled based on this treatment time under the operating conditions. The treatment time cannot be determined definitely as it varies with the operating conditions. Usually, it often falls within a range of 2 to 10 hours, particularly 3 to 8 hours, at a temperature of not less than 1200°C.

### Crystallization:

The heating is continued even after the reduction and nitridation, and allows the amorphous boron nitride to be crystallized at a temperature of not less than 1700°C, particularly in a range of 1700°C to 2200°C, resulting in a hexagonal boron nitride powder.

The temperature and holding time of the heat treatment can be adjusted appropriately in accordance with the desired diameter of the boron nitride particles. When the heat treatment is performed at a lower temperature for a shorter time, the resultant boron nitride particles tend to have a smaller diameter. When the heat treatment is performed at a higher temperature for a longer time, the resultant boron nitride particles tend to have a larger diameter. The heat treatment time is usually 0.5 to 6 hours, preferably 1 to 4 hours.

The nitrogen atmosphere in the nitriding furnace can be formed by a known means. The gas to be introduced into the nitriding furnace is not particularly limited as long as it is capable of supplying nitrogen to boron under the aforementioned nitriding treatment conditions. Examples include a nitrogen gas, an ammonia gas, and a mixed gas of a nitrogen gas or an ammonia gas and a non-oxidizing gas such as hydrogen, argon, or helium.

The nitriding furnace may be any known nitriding furnace capable of controlling the reaction atmosphere, such as an atmosphere control type high-temperature furnace in which a heat treatment is performed by high-frequency induction heating or heater heating. Alternatively, in addition to a batch furnace, a continuous furnace such as a pusher type tunnel furnace or a vertical reaction furnace is also available.

### Acid washing:

Since the hexagonal boron nitride powder obtained as described above contains, as impurities, compounds such as calcium borate, it is washed with acid, resulting in a hexagonal boron nitride powder of high purity and crystallinity. When the compounds such as calcium borate remain in large amounts, they hinder the effect of a modification treatment performed by heating as described below, which makes it difficult to obtain a boron nitride powder with few lumps.

The method of acid washing is not particularly limited, and a known method may be used. For example, the impurity-containing boron nitride powder or aggregate obtained after the nitriding treatment is put into a container by hand, to which diluted hydrochloric acid (HCl of 10 to 20 mass%) is added in an amount 5 to 10 times as much as that of the impurity-containing boron nitride powder, so that they are in contact with each other for not less than 4 hours. The acid for use in the acid washing is not limited to hydrochloric acid and may be nitric acid, sulfuric acid, acetic acid or the like.

The acid washing is followed by washing with pure water in the usual manner for removing remaining acid. Water washing is carried out in the following manner: After the acid used in the acid washing is filtered, the acid-washed boron nitride powder is dispersed in the same amount of pure water as the acid used, followed by filtration again; and the washing with pure water and the filtration are repeated until the filtrate is neutralized.

The acid washing and the water washing are followed by drying. Drying can be carried out in the atmosphere at a temperature of 50°C to 250°C or under reduced pressure. The drying is preferably carried out until the moisture content is reduced to almost 0%, and it is usually advised to carry out the drying for 1 to 48 hours at the aforementioned temperature.

The boron nitride powder from which the impurities have been removed by the acid washing and the water washing as described above is subjected to treatments of crushing, pulverization, classification and the like as needed in a known manner, followed by a modification treatment.

### <Modification treatment of boron nitride powder>

The boron nitride powder obtained as described above can be controlled so that the shape (e.g., the average long diameter, the average thickness, and the aspect ratio) of primary particles is suitable for cosmetic use, by adjusting the reaction conditions and the like. However, this boron nitride powder is lumpy and heavy in texture . In the present invention, the following modification treatment is performed, so that the boron nitride powder is modified to be fluffy (lightweight) with remarkably reduced lumps.

The modification treatment of the present invention is performed by heat treating the boron nitride powder at a temperature of 1300°C to 2200°C. It is important that this heat treatment is performed under the flow of an inert gas.

More specifically, the heat treatment (i.e., the modification treatment) is performed in the following manner: The boron nitride powder is placed under the flow of an inert gas heated to a predetermined temperature, so that the flowing inert gas comes in contact with the boron nitride powder. This treatment results in a fluffy and less lumpy boron nitride powder.

By the heat treatment under the flow of an inert gas, a slight amount of the functional groups and/or impurities present on the surface of particles of the powder are thermally decomposed, and thermolysis decompositions are removed. Accordingly, the triboelectric chargeability of the powder is changed, resulting in a fluffy and less lumpy modified boron nitride powder with a voltage density of not less than +1 V/g as measured by a triboelectric charging test under predetermined conditions, and an avalanche energy of not more than 40 mJ/kg.

The inert gas for use in the modification treatment (heat treatment) is typically a rare gas such as helium, neon, or argon. The inert gas may also be nitrogen, which is preferable in economic terms. Alternatively, a mixture of these gases is also available.

The conditions of the inert gas flow are not particularly limited as long as the gas present in the vicinity of the surface of the boron nitride powder can be diffused and then partially discharged. For example, the inert gas may flow in such a manner that it is fed onto the layer of the boron nitride powder and then discharged. Alternatively, the inert gas may flow in such a manner that it is partially replaced (fed and discharged) while fluidizing the boron nitride powder (i.e., a so-called fluidized bed). Among them, it is preferable to fluidize the boron nitride powder for efficient modification.

The amount of the inert gas flow cannot be determined definitely as it varies with the way and temperature of the heat treatment, the internal structure of the device, and the like. The flow amount is suitably 0.02 to 5 L (volume at 25°C)/min per liter volume of the boron nitride powder to be treated. In a case where a fluidized bed is used to allow the boron nitride powder to be fluidized, the treatment can be performed satisfactorily even with a smaller amount of the inert gas flow, which is specifically 0.02 to 0.5 L (volume at 25°C)/min, particularly 0.05 to 0.3 L (volume at 25°C)/min, per liter volume of the boron nitride powder to be treated.

The temperature of the heat treatment is 1300°C to 2200°C as mentioned above. When the temperature is too low, lumps cannot be reduced sufficiently, and the treatment takes a long time. When the temperature is too high, heat energy and the device cost are increased, resulting in an economic disadvantage. On this account, the heating temperature is preferably 1400°C to 2000°C, more preferably 1500°C to 1950°C. Further, the heating holding time, which varies with the treatment conditions of the inert gas and the heating temperature, may be set so that the powder has a voltage density (triboelectric charge amount) of not less than +1 V/g, preferably not less than +10 V/g, and more preferably not less than +30 V/g. Usually, the heating holding time is approximately 0.2 to 20 hours, particularly 0.5 to 10 hours.

The modification treatment can be performed by using a known device capable of controlling the reaction atmosphere, such as an atmosphere control type high-temperature furnace in which a heat treatment is performed by high-frequency induction heating or heater heating, as used for the above-described reduction and nitrization. In addition to a batch furnace, a continuous furnace such as a pusher type tunnel furnace or a vertical reaction furnace is also available.

The modification treatment of the present invention can adjust the degree of pigmentation of the powder. More specifically, when many nitrogen vacancies are generated in the boron nitride, the whiteness of the boron nitride decreases, while the yellowness increases, resulting in a modified boron nitride powder suitable for cosmetics with a color close to human skin tone. For example, in the case of using a rare gas as the inert gas, when the heating temperature is set to a range of 1700°C to 2200°C, the yellowness (b value) in the Lab color system is as high as not less than 4, resulting in a modified boron nitride powder with a color close to human skin tone.

On the other hand, in order to obtain a modified boron nitride powder with higher whiteness, a nitrogen gas may be used, and the heating temperature may be suitably set to a range of not less than 1300°C and less than 2200°C, particularly to a range of 1300°C to 2000°C, more preferably to a range of 1500°C to 2000°C, and most suitably to a range of not more than 1600°C. This results in a modified boron nitride powder with higher whiteness having a whiteness (L value) in a range of not less than 99.0, a redness (a value) in a range of -0.5 to 0, and a yellowness (b value) in a range of 0 to 2.0 in the Lab color system.

The modified boron nitride powder obtained as described above is suitable for cosmetics and capable of significantly improving spreadability and adherence to human skin by being blended in cosmetics.

### Examples

Hereinafter, the present invention will be described in more detail by way of Examples; however, the present invention is not limited to these Examples.

In Examples and Comparative Examples below, various tests and measurements were performed in the following manner.

### (1) Average long diameter, average thickness, aspect ratio

10 parts by mass of a boron nitride powder was dispersed in 100 parts by mass of an epoxy resin (EA E-30CL manufactured by Henkel Japan Ltd.) The resultant resin composition was defoamed under reduced pressure, and poured into a 10×10 mm mold with a thickness of 1 mm, followed by curing at a temperature of 70°C.

Then, the cured resin composition was removed from the mold, and subjected to polish both surfaces so that the both surfaces were parallel to each other. Thereafter, one of the surfaces perpendicular to the direction of the thickness of the resin composition was subjected to cross section milling at its center, and an image of the milled surface was taken with a SEM at a magnification of 2500.

From among the obtained images, 100 boron nitride particles were randomly chosen, and the long side (= long diameter) and the short side (= thickness) of the particles were measured in consideration of the magnification, and their average values, i.e., the average long diameter (µm) and the average thickness (µm), were calculated. Further, the aspect ratio (average long diameter/average thickness) was calculated based on these average values.

### (2) Median diameter (D1: µm)

0.3 g of a boron nitride powder was put in 50 cc of ethanol to prepare a boron nitride suspension. For this boron nitride suspension, the particle size distribution was measured by a laser diffraction scattering type particle size distribution measurement device (LA-950V2 manufactured by HORIBA, Ltd.) The obtained volume-based average particle diameter (D₅₀) was defined as the median diameter (D1).

### (3) Median diameter (D2: µm)

0.3 g of a boron nitride powder, together with 50 cc of ethanol, was put into a screw tube bottle with a capacity of 100 cc and a diameter of 4 cm to prepare a boron nitride suspension. A probe with a diameter of 0.2 cm was submerged in the water to a depth of 1 cm, and emitted ultrasonic waves at 100 W output power at room temperature for 20 minutes. For this boron nitride suspension, the particle size distribution was measured in the same manner as for the median diameter (D1). The obtained volume-based average particle diameter (D₅₀) was defined as the median diameter (D2).

### (4) Whiteness, redness, yellowness in Lab color system

Whiteness (L value), redness (a value), and yellowness (b value) were measured by using ZE 6000 manufactured by NIPPON DENSHOKU INDUSTRIES Co., LTD.

The measurement was performed by filling a quartz glass cell having a diameter of 30 mm and a height of 13 mm with a boron nitride powder.

### (5) Light bulk density, tapped bulk density

Light bulk density (g/cm³) and tapped bulk density (g/cm³) were measured by using Tap Denser KYT-5000 manufactured by SEISHIN ENTERPRISE Co., Ltd.

The measurement was performed under the following conditions: a sample cell of 100 ml, a tap speed of 120 times/min, a tap height of 5 cm, and a tapping frequency of 500 times.

### (6) Amount of eluted boron

Eluted boron was extracted in a manner in accordance with the Japanese Standards of Quasi-drug Ingredients 2006, and the amount (ppm) of boron was measured by using an ICP optical emission spectrometer.

More specifically, 2.5 g of a boron nitride powder was put in a Teflon (registered trademark) beaker, to which 10 mL of ethanol was added and stirred well. Further, 40 mL of water was added and stirred well, followed by heating at 50°C for 1 hour with a Teflon (registered trademark) watch glass placed over the beaker.

The resultant on the watch glass was cooled, followed by filtration. A residue was washed with a small amount of water, and the wash liquid and the filtrate were mixed together. The mixed liquid was further filtered through a membrane filter (0.22 µm). The entire filtrate was put in a Teflon (registered trademark) beaker, to which 1 mL of sulfuric acid was added, followed by boiling on a hot plate for 10 minutes.

The resultant liquid was cooled and then put in a polyethylene measuring flask. The Teflon (registered trademark) beaker was washed with a small amount of water, which was then added to the polyethylene measuring flask, and water was added thereto to make exactly 50 mL. The thus-obtained sample solution was subjected to measurement of the amount of boron contained therein by using an ICP optical emission spectrometer.

### (7) Voltage density (V/g)

A triboelectric charging test was performed by using, as a rotating drum type powder flowability measurement device, REVOLUTION manufactured by Mercury Scientific Inc., thereby measuring voltage density (V/g).

More specifically, 100 cc of a boron nitride powder was put in a standard rotating drum and destaticized by an ionizer. Then, the charge amount (V) of the boron nitride powder was measured while the rotating drum was rotating at a rotation frequency of 10 rpm for 300 seconds.

The charge amount (V) varies extremely unstably immediately after the rotating drum starts rotating. In light of this, the average of stable charge amounts (V) while the rotating drum was rotating for 200 to 300 seconds was calculated. This average value was divided by the weight (g) of the boron nitride powder put in the rotating drum to obtain voltage density (V/g).

The standard rotating drum has the shape of a cylinder with an inner surface made of alumite treated aluminum, includes windows made of boron silicate glass on both surfaces of the cylinder, and has a capacity of 332 cc.

### (8) Avalanche energy

A flowability test was performed by using the powder flowability measurement device as used for measuring the charge density, thereby measuring avalanche energy (mJ/kg).

More specifically, 100 cc of a boron nitride powder was put in the standard rotating drum and destaticized by an ionizer. Then, the avalanche energy (change in potential energy before and after an avalanche) (mJ/kg) of the boron nitride powder generated when the rotating drum was rotated at a rotation frequency of 0.3 rpm was measured. Here, the average of the measured values for 150 avalanches was defined as the avalanche energy (mJ/kg).

### (9) Height of powder layer in fluidization test

A fluidization test was performed by using the powder flowability measurement device, thereby measuring the height (cm) of the powder layer.

More specifically, 100 cc of a boron nitride powder was put in the standard rotating drum and destaticized by an ionizer. Then, the height (cm) (of a lower portion) of the powder layer was measured when the rotating drum was rotated at predetermined rotation frequencies (20, 50 rpm).

The height (cm) of the powder layer, which is an indicator of the fluidizability of the powder, tends to be larger when the powder is more easily fluidized.

The results of Examples are summarized in Tables, in which "*" indicates that the height of the powder was not measured correctly because the voltage density was so high that the powder was charged and adhered to the wall surface of the drum.

### (10) Basic flowability energy in dynamic flowability test

A dynamic flowability test was performed by using FT-4 Powder Rheometer manufactured by Malvern Instruments Ltd., thereby measuring basic flowability energy (mJ).

More specifically, a 160 mL split container with a height of 89 mm on which a cylinder with a height of 51 mm was placed was filled with a boron nitride powder to a height of more than 89 mm, which was then conditioned (a blade was operated at a tip speed of 60 mm/sec and an approach angle of 5°) for four times. Thereafter, the cylinder placed on the split container was slid to level off the boron nitride powder.

Then, torque (mJ) acting on the blade was measured while it was moving at a tip speed of 100 mm/sec and an approach angle of -5° from a height of 100 mm to 10 mm from the bottom surface of the container. The torque value was defined as the basic flowability energy (mJ). The basic flowability energy (mJ), which is an indicator of the flowability of the powder, tends to be smaller when the powder was more flowable.

### <Example 1>

A mixture was prepared in accordance with the following formulation by using a ball mill:
70 g of boron oxide,
30 g of carbon black, and
10 g of calcium carbonate.

This mixture was placed in a graphite Tammann furnace and heated to a temperature of 1500°C at 15°C/min in a nitrogen gas atmosphere. The mixture was kept at 1500°C for 6 hours, followed by a reduction nitridation treatment. Then, the resultant mixture was heated to a temperature of 1800°C at 15°C/min and kept at 1800°C for 2 hours, followed by a crystallization treatment, thereby obtaining a crude hexagonal boron nitride powder.

Then, the thus-obtained crude hexagonal boron nitride powder was introduced into a polyethylene container, to which an aqueous hydrochloric acid solution (HCl of 10 mass%) was added in an amount 10 times as much as that of the crude hexagonal boron nitride, and stirred at a rotation frequency of 300 rpm for 15 hours.

After the acid washing as above, the acid was filtered. Then, the resultant powder was washed again with 300 times as much pure water as the introduced crude hexagonal boron nitride powder, the pure water having a specific resistance of 1 MΩ•cm at 25°C. Then, the powder was dehydrated by suction filtration until the filtered powder had a moisture content of not more than 50 mass%.

The powder obtained after being washed with the pure water was dried under a reduced pressure of 1 kPaA at 200°C for 15 hours, resulting in a white boron nitride powder. This boron nitride powder was lumpy.

A carbon container (inner diameter: 400 mm; inner height: 50 mm) with a surface coated with boron nitride was filled with the thus-obtained boron nitride powder. The boron nitride powder was filled at a density of 0.20 g/cm³ to a height of 45 mm. These containers were stacked in ten stages and placed in a graphite Tammann furnace with an inner capacity of 1,000 L, into which a nitrogen gas was fed in an amount of 40 L (volume at 25°C)/min. Then, the boron nitride powder was heated to a temperature of 1500°C at 15°C/min and kept at 1500°C for 4 hours, whereby the boron nitride powder was modified. The modified boron nitride powder obtained after being cooled was less lumpy.

Each of the carbon containers includes a nitrogen flow channel between the respective stages of the containers to allow nitrogen to flow above the powder layer.

The thus-obtained modified boron nitride powder was subjected to the above-described measurements (1) to (10). Table 1 shows the conditions of producing and modifying the boron nitride powder, and Table 3 shows the results of the measurements.

In Table 1, "Maximum temperature" in the section on "Reduction nitridation" refers to the maximum temperature during the entire reduction nitridation process including the crystallization process after the reduction nitridation reaction. The same applies to Tables 2 and 5 below. In Tables 1 and 2, "×" in the section on "Fluidization" represents that the inert gas was fed in a state where the powder to be modified was not fluidized but left to stand. Further, "∘" in Table 2 represents that the modification treatment was performed in a fluidized bed.

Further, the modified boron nitride powder was examined visually as to how lumpy the powder was. Table 3 also shows the results of evaluation by means of the following criteria. The same applies to Table 6.
∘: Fluffy with few lumps
Δ: Fluffy with a slight amount of lumps
×: Heavy in texture with a large amount of lumps

### <Examples 2-7, 11, Comparative Examples 1-6>

Modified boron nitride powders were prepared in the same manner as in Example 1, except that the following conditions were altered: the proportion of calcium carbonate, the maximum temperature and maximum temperature holding time during the crystallization after the reduction and nitridation, the filling density of the powder in the modification treatment (heat treatment), the type and flow amount of the inert gas, the treatment temperature, and the temperature holding time.

Tables 1 and 2 show the conditions of producing and modifying the boron nitride powders in the Examples, and Tables 3 and 4 show the results of the measurements. Table 5 shows the conditions of producing and modifying the boron nitride powders in the Comparative Examples, and Table 6 shows the results of the measurements.

### <Example 8>

A crude hexagonal boron nitride powder was obtained in the same manner as in Example 1.

Then, the obtained crude hexagonal boron nitride powder was introduced into a polyethylene container, to which an aqueous hydrochloric acid solution (HCl of 10 weight%) was added in an amount 10 times as much as that of the crude hexagonal boron nitride, and stirred at a rotation frequency of 300 rpm for 15 hours. After the acid washing as above, the acid was filtered. Then, the resultant powder was washed again with 300 times as much pure water as the introduced crude hexagonal boron nitride powder, the pure water having a specific resistance of 1 MΩ•cm at 25°C. Then, the powder was dehydrated by suction filtration until the filtered powder had a moisture content of not more than 50 weight%.

The powder obtained after being washed with the pure water was dried under a reduced pressure of 1 kPaA at 200°C for 15 hours, resulting in a white boron nitride powder. This boron nitride powder was lumpy.

In order to understand the gas flow velocity required to fluidize the boron nitride powder, a ventilation test was performed by using a powder flowability measurement device, REVOLUTION manufactured by Mercury Scientific Inc. The ventilation test was performed in the same manner as the dynamic flowability test described in section (10) above, except that air was supplied from the bottom of the 160 mL split container. Torque (mJ) acting on the blade was measured as the air flow velocity was gradually increased. A small torque change represents the fluidization start flow velocity (mm/sec) , which was 3.0 mm/sec for this boron nitride powder.

Next, a carbon container (inner diameter: 150 mm; inner height: 600 mm) with a surface coated with boron nitride was filled with 600 g (about 5 L) of the boron nitride powder. At the bottom of the container, there is a gas supply port, through which a gas is supplied into the container via a porous plate and then discharged from the top of the container.

The container filled with the boron nitride powder was placed in a graphite Tammann furnace. While a nitrogen gas was fed into the container in an amount of 0.6 L (volume at 25 °C) /min, the boron nitride powder was heated to a temperature of 1300°C at 15°C/min and kept at 1300°C for 4 hours (the nitrogen flow velocity at 1300°C was 3.0 mm/sec), whereby the boron nitride powder was modified. The modified boron nitride powder obtained after being cooled was fluffy with no lumps.

Table 2 shows the conditions of producing and modifying the boron nitride powder, and Table 4 shows the results of the measurements.

### <Examples 9, 10>

Modified boron nitride powders were obtained in the same manner as in Example 8, except that the type and flow amount of the inert gas, and the treatment temperature were altered as shown in Table 2. Table 4 shows the results of the measurements of the modified boron nitride powders.

**[Table 1]**

| | | Example 1 | Example 2 | Example 3 | Example 4 | Example 5 | Example 6 |
|---|---|---|---|---|---|---|---|
| Raw material | Boron oxide (g) | 70 | 70 | 70 | 70 | 70 | 70 |
| | Carbon black (g) | 30 | 30 | 30 | 30 | 30 | 30 |
| | Calcium carbonate (g) | 10 | 10 | 10 | 10 | 10 | 10 |
| Reduction nitridation | Maximum temperature (°C) | 1800 | 1800 | 1800 | 1800 | 1800 | 1800 |
| | Maximum temperature holding time (hr) | 2 | 2 | 2 | 2 | 2 | 2 |
| Heat treatment | Filling density (g/cm³) | 0.20 | 0.20 | 0.20 | 0.20 | 0.20 | 0.20 |
| | Type of inert gas | Nitrogen | Nitrogen | Nitrogen | Nitrogen | Argon | Argon |
| | Gas flow amount (L/min) | 40 | 40 | 10 | 40 | 40 | 40 |
| | Fluidization | × | × | × | × | × | × |
| | Treatment temperature (°C) | 1500 | 1800 | 1300 | 2000 | 1400 | 1800 |
| | Temperature holding time (hr) | 4 | 4 | 1 | 10 | 4 | 4 |

**[Table 2]**

| | | Example 7 | Example 8 | Example 9 | Example 10 | Example 11 |
|---|---|---|---|---|---|---|
| Raw material | Boron oxide (g) | 70 | 70 | 70 | 70 | 70 |
| | Carbon black (g) | 30 | 30 | 30 | 30 | 30 |
| | Calcium carbonate (g) | 10 | 10 | 10 | 10 | 20 |
| Reduction nitridation | Maximum temperature (°C) | 1800 | 1800 | 1800 | 1800 | 1900 |
| | Maximum temperature holding time (hr) | 2 | 2 | 2 | 2 | 3 |
| Heat treatment | Filling density (g/cm³) | 0.20 | 0.12 | 0.12 | 0.12 | 0.40 |
| | Type of inert gas | Argon | Nitrogen | Nitrogen | Argon | Nitrogen |
| | Gas flow amount (L/min) | 40 | 0.6 | 0.5 | 0.5 | 40 |
| | Fluidization | × | o | o | o | × |
| | Treatment temperature (°C) | 20000 | 1300 | 1800 | 1800 | 1800 |
| | Temperature holding time (hr) | 10 | 1 | 1 | 1 | 4 |

**[Table 3]**

| | | Example 1 | Example 2 | Example 3 | Example 4 | Example 5 | Example 6 |
|---|---|---|---|---|---|---|---|
| Average long diameter (µm) | | 4.3 | 4.0 | 4.1 | 4.2 | 4.2 | 4.2 |
| Average thickness (µm) | | 0.6 | 0.6 | 0.6 | 0.6 | 0.6 | 0.6 |
| Aspect ratio | | 7.2 | 6.7 | 6.8 | 7.0 | 7.0 | 7.0 |
| Median diameter D1 (µm) | | 26 | 26 | 25 | 27 | 26 | 25 |
| Median diameter D2 (µm) | | 6.9 | 7.6 | 7.0 | 7.2 | 7.1 | 7.2 |
| Light bulk density (g/cm³) | | 0.10 | 0.08 | 0.10 | 0.07 | 0.09 | 0.08 |
| Tapped bulk density (g/cm³) | | 0.31 | 0.28 | 0.32 | 0.23 | 0.30 | 0.27 |
| Amount of eluted boron (ppm) | | 19 | 18 | 20 | 15 | 20 | 18 |
| Whiteness (L value) | | 99.6 | 99.1 | 99.0 | 99.3 | 99.2 | 96.7 |
| Redness (a value) | | -0.2 | -0.3 | -0.2 | -0.1 | -0.1 | -1.6 |
| Yellowness (b value) | | 0.4 | 1.3 | 1.0 | 0.9 | 0.5 | 8.2 |
| Voltage density (V/g) | | +32 | +40 | +11 | +56 | +22 | +42 |
| Avalanche energy (mJ/kg) | | 30 | 27 | 37 | 24 | 31 | 26 |
| Height of powder layer (cm) | Rotation frequency 20 rpm | 3.4 | 3.7 | 2.5 | * | 3.2 | 3.5 |
| | Rotation frequency 50 rpm | 4.2 | 4.7 | 3.0 | * | 3.9 | 4.6 |
| Basic flowability energy (mJ) | | 81 | 77 | 88 | 76 | 80 | 79 |
| Development of lumps (visual examination) | | ∘ | o | ∘ | o | o | o |

**[Table 4]**

| | | Example 7 | Example 8 | Example 9 | Example 10 | Example 11 |
|---|---|---|---|---|---|---|
| Average long diameter (µm) | | 4.3 | 4.0 | 4.1 | 4.1 | 9.2 |
| Average thickness (µm) | | 0.6 | 0.6 | 0.6 | 0.6 | 1.1 |
| Aspect ratio | | 7.2 | 6.7 | 6.8 | 6.8 | 8.3 |
| Median diameter D1 (µm) | | 27 | 23 | 22 | 23 | 30 |
| Median diameter D2 (µm) | | 7.2 | 6.6 | 6.7 | 6.7 | 10.4 |
| Light bulk density (g/cm³) | | 0.07 | 0.08 | 0.07 | 0.07 | 0.17 |
| Tapped bulk density (g/cm³) | | 0.25 | 0.22 | 0.23 | 0.22 | 0.55 |
| Amount of eluted boron (ppm) | | 16 | 20 | 17 | 17 | 10 |
| Whiteness (L value) | | 95.8 | 99.5 | 99.4 | 93.6 | 99.3 |
| Redness (a value) | | -2.8 | -0.2 | -0.1 | -1.5 | -0.1 |
| Yellowness (b value) | | 12.3 | 0.6 | 0.8 | 14.1 | 0.8 |
| Voltage density (V/g) | | +53 | +38 | +59 | +57 | +43 |
| Avalanche energy (mJ/kg) | | 25 | 23 | 21 | 22 | 24 |
| Height of powder layer (cm) | Rotation frequency 20 rpm | * | 3.6 | * | * | 3.8 |
| | Rotation frequency 50 rpm | * | 4.7 | * | * | 4.6 |
| Basic flowability energy (mJ) | | 74 | 75 | 72 | 69 | 79 |
| Development of lumps (visual examination) | | o | ∘ | o | o | o |

**[Table 5]**

| | | Comparative Example 1 | Comparative Example 2 | Comparative Example 3 | Comparative Example 4 | Comparative Example 5 | Comparative Example 6 |
|---|---|---|---|---|---|---|---|
| Raw material | Boron oxide (g) | 70 | 70 | 70 | 70 | 70 | 70 |
| | Carbon black (g) | 30 | 30 | 30 | 30 | 30 | 30 |
| | Calcium carbonate (g) | 10 | 10 | 10 | 10 | 10 | 20 |
| Reduction nitridation | Maximum temperature (°C) | 1800 | 1800 | 1800 | 1800 | 1800 | 1900 |
| | Maximum temperature holding time (hr) | 2 | 2 | 2 | 2 | 2 | 3 |
| Heat treatment | Filling density (g/cm³) | Not performed | 0.20 | 0.20 | 0.20 | 0.20 | Not performed |
| | Type of inert gas | | Nitrogen | Nitrogen | Vacuum | Vacuum | |
| | Gas flow amount (L/min) | | 40 | 0 | 0 | 0 | |
| | Fluidization | | × | × | × | × | |
| | Treatment temperature (°C) | | 1200 | 1500 | 1500 | 1800 | |
| | Temperature holding time (hr) | | 1 | 4 | 4 | 4 | |

**[Table 6]**

| | | Comparative Example 1 | Comparative Example 2 | Comparative Example 3 | Comparative Example 4 | Comparative Example 5 | Comparative Example 6 |
|---|---|---|---|---|---|---|---|
| Average long diameter (µm) | | 4.0 | 4.1 | 4.1 | 4.2 | 4.1 | 9.2 |
| Average thickness (µm) | | 0.6 | 0.6 | 0.6 | 0.6 | 0.6 | 1.1 |
| Aspect ratio | | 6.7 | 6.8 | 6.8 | 7.0 | 6.8 | 8.3 |
| Median diameter D1 (µm) | | 23 | 24 | 25 | 27 | 25 | 29 |
| Median diameter D2 (µm) | | 6.7 | 7.0 | 7.0 | 6.8 | 7.3 | 10.1 |
| Light bulk density (g/cm³) | | 0.12 | 0.12 | 0.11 | 0.12 | 0.10 | 0.21 |
| Tapped bulk density (g/cm³) | | 0.35 | 0.34 | 0.32 | 0.35 | 0.30 | 0.69 |
| Amount of eluted boron (ppm) | | 8 | 20 | 20 | 19 | 18 | 5 |
| Whiteness (L value) | | 98.8 | 98.7 | 98.2 | 98.9 | 96.4 | 98.5 |
| Redness (a value) | | -0.3 | -0.2 | -0.4 | -0.3 | -1.1 | -0.2 |
| Yellowness (b value) | | 1.6 | 1.4 | 2.1 | 1.8 | 4.1 | 1.9 |
| Voltage density (V/g) | | -34 | -12 | -6 | -4 | -1 | -28 |
| Avalanche energy (mJ/kg) | | 48 | 45 | 45 | 44 | 42 | 43 |
| Height of powder layer (cm) | Rotation frequency 20 rpm | 1.7 | 1.6 | 2.1 | 1.9 | 2.2 | 1.5 |
| | Rotation frequency 50 rpm | 1.9 | 2.1 | 2.6 | 2.5 | 2.8 | 1.8 |
| Basic flowability energy (mJ) | | 203 | 195 | 161 | 174 | 140 | 218 |
| Development of lumps (visual examination) | | × | × | Δ | Δ | Δ | × |

### [Cosmetics test]

A powder foundation was prepared by using the modified boron nitride powder obtained in each of the Examples and Comparative Examples in accordance with the following blending proportions (here, for the modified boron nitride powder in each of Examples 6, 7, 10, and Comparative Example 5, the proportions of a red oxide of iron, a yellow oxide of iron, and a black oxide of iron were properly adjusted to prepare a powder foundation whose color was identical to that of a powder foundation using the other white boron nitride.)

| | |
|---|---|
| Hexagonal boron nitride powder | 20.0 mass% |
| Mica | 15.0 mass% |
| Synthetic phlogopite | 12.0 mass% |
| Ethylhexyl methoxycinnamate | 8.0 mass% |
| (Vinyl dimethicone/methicone silsesquioxane crosspolymer) | 8.0 mass% |
| (Diphenyl dimethicone/vinyl diphenyl dimethicone/silsesquioxane crosspolymer) | 8.0 mass% |
| Nylon 12 | 3.0 mass% |
| Silica | 3.0 mass% |
| Talc | 3.0 mass% |
| Acrylates crosspolymer | 3.0 mass% |
| Perfluorooctyl triethoxysilane | 3.0 mass% |
| Zinc oxide | 3.0 mass% |
| Polymethyl methacrylate polymer | 3.0 mass% |
| Silicone treated red iron oxide (red oxide of iron) | 1.0 mass% |
| Silicone treated yellow iron oxide | 0.6 mass% |
| Silicone treated black iron oxide | 0.4 mass% |
| Silicone treated titanium oxide | 6.0 mass% |

The obtained powder foundation was taken onto a cosmetic sponge and applied to skin. The foundation using the modified boron nitride powder visually examined as "o" in Tables 3 and 4 was spread evenly in one application, while the foundation using the modified boron nitride powder visually examined as "Δ" or "x" in Table 6 was spread unevenly in one application and had to be applied two or three times until it was spread evenly.

Further, the powder foundation using the modified boron nitride powder whose color was close to human skin tone as in Examples 6, 7, 10, and Comparative Example 5 had a more natural finish than the powder foundation using the other modified boron nitride powder with higher whiteness.

## Claims

1. A modified boron nitride powder having a voltage density of not less than +1 V/g as measured by a triboelectric charging test.

2. The modified boron nitride powder according to claim 1, having a voltage density of not less than +10 V/g as measured by a triboelectric charging test.

3. The modified boron nitride powder according to claim 1, having an avalanche energy of not more than 40 mJ/kg.

4. The modified boron nitride powder according to claim 1, containing primary particles with an average long diameter in a range of 2 to 20 µm, an average thickness in a range of 0.2 to 2.0 µm, and an average aspect ratio in a range of 5 to 20.

5. The modified boron nitride powder according to claim 1, for use as a compounding agent for cosmetics.

6. A method for modifying a boron nitride powder, comprising: heat treating a negatively triboelectrically charged boron nitride powder under a flow of an inert gas at a temperature of 1300°C to 2200°C until it has a voltage density of not less than +1 V/g as measured by a triboelectric charging test.

7. The method according to claim 6, wherein the heat treatment is performed until the negatively triboelectrically charged boron nitride powder has a voltage density of not less than +10 V/g as measured by the triboelectric charging test.

8. The method according to claim 6, wherein the heat treatment is performed while the negatively triboelectrically charged boron nitride powder is fluidized under the flow of the inert gas.
